(19) Europäisches Patentamt
European Patent Office
Office européen des brevets

(11) Publication number: **0 027 010**
**B1**

# (12) EUROPEAN PATENT SPECIFICATION

(45) Date of publication of patent specification: **05.10.83**

(51) Int. Cl.[3]: **C 07 D 499/04**

(21) Application number: **80303379.4**

(22) Date of filing: **26.09.80**

## (54) Process for the preparation of penam derivatives.

(30) Priority: **09.10.79 GB 7934994**

(43) Date of publication of application:
**15.04.81 Bulletin 81/15**

(45) Publication of the grant of the patent:
**05.10.83 Bulletin 83/40**

(84) Designated Contracting States:
**CH IT LI NL SE**

(56) References cited:
**GB - A - 1 401 059**
**US - A - 3 954 731**

(73) Proprietor: **BEECHAM GROUP PLC**
**Beecham House Great West Road**
**Brentford Middlesex (GB)**

(72) Inventor: **Lashford, Andrew Gerard**
**60 Sydney Road**
**Sutton Surrey (GB)**

(74) Representative: **Hesketh, Alan, Dr. et al,**
**European Patent Attorney Beecham**
**Pharmaceuticals Great Burgh Yew Tree Bottom**
**Road**
**Epsom, Surrey, KT18 5XQ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 027 010 B1

Courier Press, Leamington Spa, England

Process for the preparation of penam derivatives

This invention relates to a process for the preparation of antibacterially active penam derivatives having a 6$\alpha$-methoxy substituent from compounds having a 6$\alpha$-arylthio derivatives.

British Patent Specification No. 1,439,898 discloses *inter alia* a process for preparing a 6$\alpha$-alkoxy-6$\beta$-acylamino penam derivative by treating a corresponding 6$\alpha$-alkylthio-6$\beta$-acylamino penam derivative with an alkyl alcohol in the presence of a silver ion. There is no disclosure in that specification of an acylamino group having a carboxylic acid moiety therein. The present invention is based on the fact that when the penam derivative contains a carboxy group in the acylamino side-chain, a 6$\alpha$-arylthio penam derivative may be advantageously employed for the preparation of a corresponding 6$\alpha$-methoxy derivative.

Accordingly the present invention provides a process for the preparation of a penam derivative of formula (I):

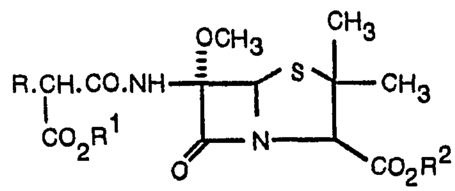

(I)

wherein R is phenyl, *p*-hydroxyphenyl, p-($C_{1-6}$)-alkylcarbonyloxyphenyl or 2- or 3-thienyl, $R^1$ is hydrogen, or a pharmaceutically acceptable salt-forming ion or ester-forming radical, and $R^2$ represents hydrogen or a pharmaceutically acceptable salt-forming ion or *in vivo* hydrolysable ester-forming radical; characterised by reacting a compound of formula (II):

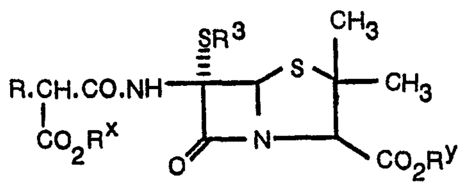

(II)

wherein R is as defined with respect to formula (I) above, $R^x$ represents an ester-forming radical, $R^y$ represents hydrogen, a salt-forming radical or a carboxyl-blocking group, and $R^3$ represents an aryl group; with methanol in the presence of either: (a) silver, thallium or mercuric ions; or (b) chlorine and a base; and thereafter if necessary carrying out one or more of the following steps:

(i) removal of any carboxyl blocking group;

(ii) converting the product to a pharmaceutically acceptable salt or ester thereof.

A suitable p-($C_{1-6}$)alkylcarbonyloxyphenyl group for the group R is p-acetoxyphenyl.

Suitable pharmaceutically acceptable salt-forming ions for the groups $R^1$ and $R^2$ include metal salts, eg aluminium, alkali metal salts such as sodium or potassium, alkaline earth metal salts such as calcium or magnesium, and ammonium or substituted ammonium salts, for example those with lower alkylamines such as triethylamine, hydroxy-lower alkylamines such as 2-hydroxyethylamine, bis-(2-hydroxyethyl)amine or tri-(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-$\beta$-phenethylamine, dehydroabeitylamine, N,N'-bisdehydroabietylenediamine, or bases of the pyridine type such as pyridine, collidine or quinoline, or other amines which have been used for form salts with known penicillins.

The salt-forming ions included within the definition of the group $R^y$ include the above mentioned ions and also include other salt-forming ions which are not necessarily pharmaceutically acceptable.

When the group $R^2$ represents a pharmaceutically acceptable *in vivo* hydrolysable ester-forming radical, such esters are those which hydrolyse readily in the human body to produce the parent acid, and include, for example, acyloxyalkyl groups such as acetoxymethyl, pivaloyloxymethyl, $\alpha$-acetoxyethyl, $\alpha$-acetoxybenzyl and $\alpha$-pivaloyloxyethyl groups; alkoxycarbonyloxyalkyl groups, such as ethoxycarbonyloxymethyl and $\alpha$-ethoxycarbonyloxyethyl; dialkylaminoalkyl groups such as dimethylaminomethyl, dimethylaminoethyl, diethylaminomethyl or diethylaminoethyl; and lactone groups such as phthalidyl or dimethoxyphthalidyl.

The group $R^1$ may be any of the ester-forming radicals as specified for the group $R^2$ and in addition $R^1$ may represent other pharmaceutically acceptable ester-forming groups such as alkyl, aryl or aralkyl groups any of which may be substituted. Examples of such groups include:

a) $C_{1-6}$ alkyl such as methyl, ethyl, *n*- and *iso*-propyl, *n*-, *sec*-, and *tert*-butyl;

b) substituted $C_{1-6}$ alkyl wherein the substituent is at least one of: chloro, bromo, fluoro, nitro, $C_{1-6}$

alkoxy, $C_{1-6}$ alkoxycarbonyl, cyano, $C_{1-6}$ alkylthio, $C_{1-6}$ alkylamino;
c) phenyl, benzyl or substituted phenyl or benzyl wherein the substituent is at least one of chloro, bromo, fluoro, $C_{1-6}$ alkyl, $C_{1-6}$ alkoxy, $C_{1-6}$ alkanoyl, $C_{1-6}$ alkoxycarbonyl, nitro or di-($C_{1-6}$)alkylamino.

Preferred ester-forming radicals $R^1$ include $C_{1-6}$ alkyl, benzyl, phthalidyl, indanyl, phenyl and mono-, di-, and tri-($C_{1-6}$)-alkyl substituted phenyl such as *o-*, *m-*, or *p*-methylphenyl, ethylphenyl, *n-* or *iso*-propylphenyl, or *n-*, *sec-*, *iso- or t*-butylphenyl.

Suitable carboxyl-blocking groups for the group $R^y$ are those which may be readily removed from the carboxylic acid under conventional conditions at a later stage of the reaction. Such groups include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula —N=CHR° where R° is aryl or heterocyclic, or an *in vivo* hydrolysable ester radical such as defined above.

The carboxyl group may be regenerated from any of the above esters by usual methods appropriate to the particular $R^y$ group, for example, acid—and base—catalysed hydrolysis, or by enzymically—catalysed hydrolysis, or by hydrogenation.

When it is desired to produce a compound of formula (I) wherein the group $R^1$ is hydrogen or a salt-forming ion, by the process of this invention, a compound of formula (II) is employed wherein $R^x$ is a carboxyl-blocking group. For the preparation of a compound of formula (I) wherein $R^1$ is a pharmaceutically acceptable esterforming radical, a compound of formula (II) is employed wherein $R^x$ represents the desired $R^1$ group.

When the group R represents p-hydroxyphenyl, it may if desired be protected by means of a group which is readily removed chemically after the process of the invention. Such protecting groups include trialkylsilyl groups.

Suitable examples of the aryl group $R^3$ include phenyl, optionally substituted with $C_{1-6}$ alkyl, $C_{1-6}$ alkoxy, halogen, or nitro. Preferred groups for $R^3$ include phenyl, *o-*, m- or *p*-methylphenyl, *o-*, *m-* or *p*-nitrophenyl.

A suitable temperature range for the process of this invention is from −20°C to +20°C, conveniently −5°C to +5°C. The time required for the reaction depends on the temperature and the reagents employed. Generally, the reaction is complete within one hour and usually within 5 to 10 minutes. The methanol used in the process is conveniently employed as a solvent for the reaction mixtures. Other compatible co-solvents may be additionally used if desired.

Suitable sources of mercuric ion include for example, mercuric chloride, mercuric acetate and mercuric trifluoroacetate. Suitable silver salts include the nitrate benzoate and acetate. A suitable thallium salt is thallium nitrate.

The starting material for the process of this invention, i.e. compound of formula (II) above, is disclosed, although no claimed, in US Patent No. 3,965,093. It may be prepared by acylation, under conventional conditions of a 6-amino compound of formula (III) or a salt or ester thereof:

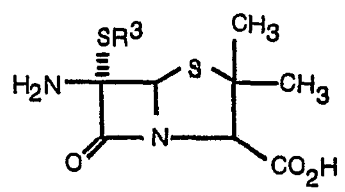

(III)

wherein $R^3$ is as defined with respect to formula (II) above. Compounds of formula (III) may be prepared from a Schiff's base derivative as described in US Patent No. 3,965,093, or may be prepared by reacting a thiooxime compound of formula (IV):

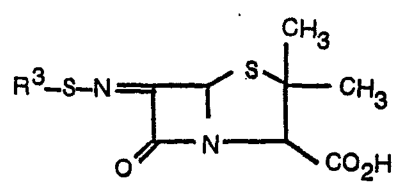

(IV)

(wherein $R^3$ is as defined with respect to formula (II) above) with a tri(alkyl)phosphine or tri(aryl)phosphine, followed by treatment with an acid catalyst such as silica gel. That process is described in US Patent No. 4,119,778 and in J. Amer Chem Soc, 1977, *99*, 5504.

The compounds of formula (I) which are prepared by the process of this invention have good antibacterial activity, as disclosed in British Patents Nos. 1,538,051 and 1,538,052.

The following Examples illustrate the process of this invention.

Example 1

Method of preparation of 6,β-(2-carboxy-2-thien-3′-ylacetamido)-6,α-methoxypenicillanate

a) Benzyl 6 - β - [2 - (4 - methylphenoxycarbonyl) - 2 - thien - 3′ - ylacetamido] - 6,α - (4 - methylphenylthio)penicillanate

To benzyl 6,β-amino-6,α-(4-methylphenylthio)penicillanate (prepared by adaptation of the method reported for the 2,2,2-trichloroethyl ester, J Amer Chem Soc, 1977, *99*, 5504) (233 mg) and pyridine (80 μl) in dichloromethane (10 ml) at 0°C was added 2-(4-methylphenoxycarbonyl)-2-thien-3′-ylacetyl chloride (1 equivalent) in dichloromethane (10 ml). After 5 minutes at room temperature the solution was washed with water, dilute sodium bicarbonate solution, dilute hydrochloric acid, water, and brine then dried, evaporated and the residue chromatographed on silica to give the title compound, 290 mg, 78% yield, δ ($CDCl_3$) 1.30 (6H, bs, 2 x $2CH_3$), 2.30 (6H, m, 2 x Ar*$CH_3$*), 4.37, 4.38 (1H, 2 x s, 3H), 4.83 (1H, s, *CH*CONH), 5.14 (2H, s, O*$CH_2$*Ph), 5.58 (1H, s, 5H), 6.8—7.6 (17H, m, Ph, 2 x $C_6H_4$, thienyl and CONH).

b. Benzyl 6,β-[2-(4-methylphenoxycarbonyl)-2-thien-3′-ylacetamido]-6,α-methoxypenicillanate

Benzyl 6,β-[2-(4-methylphenoxycarbonyl)-2-thien-3′-ylacetamido]-6,α(4-methylphenylthio)-penicillanate (155 mg) in methanol (15 ml) at 0°C was treated with mercuric acetate (75 mg) in methanol (5 ml). After 5 minutes the solvent was removed *in vacuo*, the residue dissolved in chloroform washed three times with water, dried, evaporated and the residue chromatographed on silica to give the title compound 91 mg, 68% yield, $\nu_{max}$ ($CHCl_3$) 1760, 1730 and 1660 $cm^{-1}$, δ ($CDCl_3$ 1.30 (6H, m, 2 x $2CH_3$), 2.28, 2.30 (3H, 2 x s), Ar*$CH_3$*, 3.39, 3.42 (3H, 2 x s, $OCH_3$), 4.39, 4.41 (1H, 2 x s, 3H), 4.95 (1H, s, *CH*CONH), 5.16 (2H, s, O*$CH_2$*Ph), 5.57 (1H, s, 5H), 6.9—7.8 (13H, m, Ph, $C_6H_4$, thienyl and CONH).

c) 6,α-Methoxy-6,β-[D,L-2-(4-methylphenoxycarbonyl)-thien-3-ylacetamido]penicillanic acid

Benzyl 6,α-methoxy-6,β-[D,L-2-(4-methylphenoxycarbonyl)-thien-3-ylacetamido]penicillanate (1.2 gm) was dissolved in distilled ethanol (60 ml) and treated with water (6 ml). Palladium on carbon 10% (0.40 gm) was added and the mixture hydrogenated for 1 hour. The mixture was filtered through Celite, the residue being washed with distilled ethanol (5 ml). Further catalyst (0.40 gm) was added and hydrogenation continued until removal of benzyl ester was complete as shown by tlc on silica gel in chloroform/acetone/acetic acid in 50:50:7. Water (25 ml) was added and the ethanol removed *in vacuo*. The mixture was made just alkaline with sodium bicarbonate solution, washed with ether and acidified with 5*N* hydrochloric acid. Extraction with ether (2 x 25 ml), washing of the extracts with water (2 x 10 ml), drying and evaporation *in vacuo* gave the title compound as its free acid. Tlc Rf = 0.65 ($SiO_2$: chloroform/acetone/acetic acid—50:50:7). Nmr ($CDCl_3$) 6 = 1.40 (6H, m, gem-dimethyls), 2.38 (3H, s, tolyl —*$CH_3$*), 3.52 (3H, s, —O*$CH_3$*), 4.51 (1H, s, C—3 proton). 5.17 (1H, s,

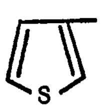 *CH*<),

5.71 (1H, s, C—5 proton), 7.0—7.75 (5H, m, tolyl aromatics and thienyl 4-proton), 8.18 (3H, m, thienyl 2- and 5-protons, —CO*NH*—). This material was dissolved in dry ether and treated with one equivalent of sodium 2-ethylhexoate (as a *ca* 2*M* solution in 4-methylpentan-2-one) diluted with dry ether. The solid was filtered, washed with ether and dried to give the required compound as its sodium salt in 16% yield. $\nu_{max}$ (KBr) : 3410 (broad), 2962, 1758, 1684, 1602, 1504, 1402, 1335, 1196, 1167, 1130, 848 and 779 $cm^{-1}$.

d) 6,α-Methoxy-6,β-(D,L-2-carboxy-2-thien-2′-ylacetamido)penicillanic acid

The α-(4-methylphenyl) ester described in c) above was hydrolysed with sodium tetraborate decahydrate in water by the procedure described in Example 4 of British Patent Specification No. 1,538,052 to give the title product: NMR [$(CD_3)_2CO$], δ = 1.50 (6H, m, gem-dimethyls), 3.52 (3H, d, —O*$CH_3$*), 4.50 (1H, d, 3—H), 5.32 (1H, s, <*CH*CONH—), 5.65 (1H, s, 5—H), 7.3 (3H, m, thienyl aromatics) 8.88 (1H, d, —CON*H*—), 9.28 (2H, s, —$CO_2H$).

Example 2

a) Benzyl 6,β-(2-phenoxycarbonyl-2-thien-3′-ylacetamido)-6,α-(4-methylphenylthio)penicillanate

To a solution of benzyl 6,β-amino6,α-(4-methylphenylthio)penicillanate (23.4 g) and pyridine (10 ml) in dichloromethane (100 ml) at 0°, was added 2-phenoxycarbonyl-2-thien-3′-ylacetyl chloride (14.0 g) in dichloromethane (100 ml). After stirring for one hour at room temperature the solvents were evaporated. The residue was dissolved in ethyl acetate and washed with water, dilute hydrochloric acid, dilute sodium bicarbonate and water, dried and evaporated to a yellow foam. This was crystallised from methanol, 27.8 g, 82.7%, m.p. 105—107°, δ ($CDCl_3$) 1.32 (6H, b.s., 2 x $2CH_3$) 2.33 (3H, s, $PhCH_3$),

4.42 (1H, s, 3H) 4.91 (1H, s, *CH*CONH), 5.20 (2H, s, $CH_2$Ph) 5.66 (1H, s, 5H) 6.9—7.7 (18H, m, aromatics and CONH).

b) Benzyl 6,β-(2-carboxy-2-thien-3′-ylacetamido)-6,α-(4-methylphenylthio)penicillanate

To a solution of benzyl 6,β-amino-6,α-(4-methylphenylthio)penicillanate (8.56 g) and pyridine (4.0 ml) in tetrahydrofuran (50 ml) at 0° was added a solution of 2-thien-3′-yl-2-trimethylsilyloxycarbonylacetyl chloride (5.53 g) in diisopropyl ether (70 ml). This was stirred at room temperature for one hour, then washed with dilute hydrochloric acid and water. The organic solution was extracted with dilute sodium bicarbonate solution and, after acidification, extracted with ethyl acetate. The organic extracts were washed with water and brine, dried and evaporated to a brown foam, 10.77 g, 90.4%. δ ($CDCl_3$) 1.29 (6H, b.s., 2 × $2CH_3$) 2.28 (3H, s, Ph$CH_3$) 4.26, 4.28 (1H, 2 × s, 3H), 4.86, 4.91 (1H, s 2 s, *CH*CONH) 5.16 (2H, s, $CH_2$Ph) 5.60 (1H, s, 5H) 6.8—7.6 (12H, m, aromatics), 7.75, 7.86 (1H, 2 × b.s., CONH) 9.13 (1H, b.s., $CO_2$H).

c) Benzyl 6,β-(2-phenoxycarbonyl-2-thien-3′-ylacetamido)-6,α-(4-methylphenylthio)penicillanate

To a solution of benzyl 6,β-(2-carboxy-2-thien-3′-ylacetamido)-6,α-(4-methylphenylthio)-penicillanate (1.2 g) in dichloromethane (25 ml) at 0° was added phenol (188 mg) then dicyclohexylcarbodiimide (440 mg). After stirring at room temperature for forty-two hours the mixture was filtered and evaporated. The title compound was isolated by chromatography on silica as a foam in 89.3% yield.

d) Benzyl 6,α-methoxy-6,β-(2-phenoxycarbonyl-2-thien-3′-ylacetamido)penicillanate

Benzyl 6,α-(4-methylphenylthio-6,β-(2-phenoxycarbonyl)-2-thien-3′-ylacetamido)penicillanate (3.36 g) is isopropyl acetate (5 ml) and methanol (25 ml) was treated at 35° with a solution of silver nitrate (1.5 g) in pyridine (5 ml). This was stirred at room temperature for thirty minutes then filtered. The filtrate was washed with water, 1% aqueous sodium sulphide and water, then dried and evaporated to give the title compound as a foam, 2.4 g, 82.8%. δ ($CDCl_3$) 1.30 (6H, b.s., 2 × $2CH_2$), 3.43 (3H, s, $OCH_3$) 4.43 (1H, s, 3H), 5.01 (1H, s, *CH*CONH), 5.18 (2H, s, $CH_2$Ph), 5.60 (1H, s, 5H), 6.8—7.7 (14H, m, aromatics and CONH).

e) Benzyl 6,α-methoxy-6,β-(2-carboxy-2-thien-3′-ylacetamido)penicillanate

A solution of benzyl 6,α-methoxy-6,β-(2-phenoxycarbonyl-2-thien-3′-ylacetamido)penicillanate (5.2 g) in tetrahydrofuran (500 ml) was stirred with 0.05M sodium tetraborate solution (three equivalents) for five hours at room temperature. The tetrahydrofuran was evaporated and ether added. The aqueous layer was acidified with dilute hydrochloric acid and the layers separated. The ether was extracted with dilute sodium bicarbonate solution which was then acidified and extracted with dichloromethane. The organic extract was washed with water and brine and dried over magnesium sulphate. The solvent was evaporated to yield the title compound as a foam, 3.0 g, 66.7%, δ ($CDCl_3$) 1.30 (6H, b.s., 2 × $2CH_3$), 3.43, 3.46 (3H, 2 × s, $OCH_3$), 4.50 (1H, s, 3H), 4.92 (1H, s, C*H*CONH) 5.26 (2H, s, $CH_2$PH) 5.66 (1H, s, 5H), 6.8—7.7 (9H, m, aromatics), 8.40, 8.52 (1H, 2 × b.s., CONH), 10.17 (1H, b.s., $CO_2$H).

f) 6,α-Methoxy-6,β-(D,L-2-carboxy-2-thien-3′-ylacetamido) penicillanic acid

Benzyl 6,β-(2-carboxy-2-thien-3′-ylacetamido)-6,α-methoxypenicillanate (2.79 g) was dissolved in water (200 ml) containing sodium bicarbonate and hydrogenated in the preesence of 10% palladium on charcoal (2.5 g then 2.0 g). The filtered solution was washed with ether, acidified and extracted with ethyl acetate. The extracts were washed with water and brine, dried and evaporated to a foam, 1.06 g. T.l.c. $R_f = 0.53$ ($SiO_2$ : Butanol/ethanol/water, 12:3:5).

This was dissolved in acetone (20 ml) and treated with sodium 2-ethylhexoate (1.1 ml) of a 2*M* solution in 4-methyl-pentan-2-one). The precipitate was filtered, washed with acetone, then ether and dried, 0.66 g, 27.2%.

g) Benzyl 6,α-methoxy-6,β-(2-phenoxycarbonyl-2-thien-3′-ylacetamido)penicillanate

Benzyl 6,-(4-methylphenylthio)-6,β-(2-phenoxycarbonyl-2-thion-3′-ylacetamido)penicillanate (672 mg) in dichloromethane (80 ml) at −60° was treated with a solution of chlorine (one equivalent in dichloromethane (3.75 ml). After stirring at −60° for five minutes a solution of triethylamine (one equivalent) in methanol (5 ml) was added and the solution allowed to warm to room temperature. The presence of the title compound was shown by comparison of t.l.c. with the product of Example 2(d).

Example 3

Benzyl 6,β - [2 - (2 - methylphenoxycarbonyl) - 2 - thien - 3′ - ylacetamido] - 6,α - (4 - methylphenylthio)penicillanate

To a solution of benzyl 6,β-amino-6,α-(4-methylphenylthio)penicillanate (21.4 g) and pyridine (10 ml) in dichloromethane (100 ml) at −10° was added 2-(2-methylphenoxycarbonyl)thien-3′-ylacetyl chloride (14.7 g) in dichloromethane (100 ml). After stirring for one hour at 0°C the solvent

was evaporated and the residue dissolved in ethyl acetate. This was washed successively with water, dilute hydrochloric acid, water, dilute sodium bicarbonate, water and brine, dried and evaporated to a foam 32.0 g, 93%, δ ($CDCl_3$), 1.16 (6H, m, 2 × $2CH_3$), 2.25 (6H, b.s., 2 × Ar$CH_3$), 4.46 4.53 (1H, 2 × s, 3H), 5.21 (2H, b.s., $CH_2$Ph), 5.38 (1H, b.s., *CH*CONH), 5.72, 5.75 (1H, 2 × s, 5H), 6.7—7.7 (17H, m, aromatics) and CONH).

Example 4

a) 4 - Nitrobenzyl 6,α - (4 - methylphenylthio) - 6,β - (2 - phenoxycarbonyl - 2 - thien - 3′ - ylacetamido)penicillanate

To 4-Nitrobenzyl-6,β-amino-6,α-(4-methylphenylthio)penicillanate (1.0 g) and pyridine (0.27 ml) in dichloromethane (10 ml) at −10° was added 2-phenoxycarbonyl-2-thien-3′-ylacetyl chloride (1.19 g) in dichloromethane (10 ml). After stirring at room temperature for thirty minutes the solution was washed with water, dilute sodium bicarbonate, dilute hydrochloric acid and brine then dried over magnesium sulphate and evaporated to a yellow foam. This was chromatographed in silica to yield the title compound, 1.0 g, 66%, δ ($CDCl_3$) 1.32 (6H, b.s., 2 × $2CH_3$), 2.25, 2.29 (3H, 2 × s, Ph$CH_3$), 4.46 (1H, s, 3H), 4.90 (1H, s, *CH*CONH), 5.25 (2H, b.s., $CH_2$Ph), 5.61 (1H, s, 5H), 6.95—8.40 (17H, m, aromatic, CONH).

4-Nitrobenzyl 6,α-methoxy-6,β-(2-phenoxycarbonyl-2-thien-3′-ylacetamido)penicillanate

4-Nitrobenzyl 6,α-(4-methylphenylthio)-6,β-(2-phenoxycarbonyl-2-thien-3′-ylacetamido)-penicillanate (1.0 g) in methanol (90 ml) at 0° was treated with mercuric acetate (0.449 g) in methanol (10 ml) and stirred at 0° for five minutes. The solvent was removed and the residue dissolved in chloroform. This was washed with water, dried and evaporated. Chromatography gave the title compound as an oil, 0.4 g, 45.8%. δ ($CDCl_3$) 1.40 (6H, b.s., 2 × $2CH_3$), 3.47, 3.55 (3H, 2 × s, $OCH_3$), 4.54 (1H, s, 3H) 5.21 (1H, s, *CH*CONH), 5.35 (2H, s, $CH_2$Ph), 5.65 (1H, s, 5H), 7.1—8.5 (13H, m, aromatics, NH).

Example 5

a) Benzyl 6,β-[2-(4-methylphenoxycarbonyl) - 2 - thien - 3′ - ylacetamido] - 6,α - phenylthio-penicillanate

To a solution of benzyl 6,β-amino-6,α-phenylthiopenicillanate (42 mgs) and pyridine (15 μl) in dichloromethane at 0°C was added 2-(4-methylphenoxycarbonyl)-2-thien-3′ylacetyl chloride (43 mg). This was allowed to come to room temperature and stirred for five minutes. The solution was washed with water, dilute hydrochloric acid, dilute sodium bicarbonate, water and brine, dried and evaporated to an oil, 60 mg, 85%. δ ($CDCl_3$) 1.31 (6H, b.s., 2 × $2CH_3$(, 2.37 (3H, s, Ph$CH_3$), 4.49 (1H, b.s., 3H), 5.00 (1H, s, *CH*CONH), 5.26 (2H, s, $CH_2$Ph), 5.72 (1H, s, 5H), 6.9—7.9 (18H, m, aromatics and NH).

b) Benzyl 6,α - methoxy - 6,β - [2 - (4 - methylphenoxycarbonyl) - 2 - thien - 3′ - ylacetamido]-penicillanate

Benzyl 6,β-[2-(4-methylphenoxycarbonyl)-2-thien-3′-ylacetamido]-6,α-phenylthiopenicillanate (42 mg) in methanol (10 ml) was treated at room temperature with mercuric acetate (one equivalent) in methanol (2 ml). After stirring for five minutes the presence of the title compound was shown by t.l.c. and h.p.l.c. comparison with the product of Example 1(b).

**Claims**

1. A process for the preparation of a penam derivative of formula (I):

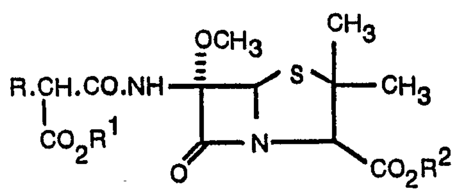

(I)

wherein R is phenyl, *p*-hydroxyphenyl, p-($C_{1-6}$)-alkylcarbonyloxyphenyl or 2- or 3-thienyl, $R^1$ is hydrogen, or a pharmaceutically acceptable salt-forming ion or ester-forming radical, and $R^2$ represents hydrogen or a pharmaceutically acceptable salt-forming ion or *in vivo* hydrolysable ester-forming radical; characterised by reacting a compound of formula (II):

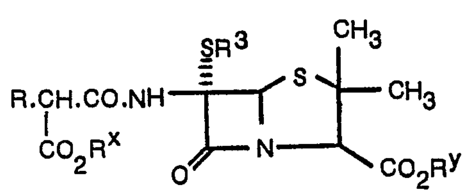

(II)

wherein R is as defined with respect to formula (I) above, $R^x$ represents an ester-forming radical, $R^y$ represents hydrogen, a salt-forming radical or a carboxyl-blocking group, and $R^3$ represents an aryl group; with methanol in the presence of either: (a) silver, thallium or mercuric ions; or (b) chlorine and a base; and thereafter if necessary carrying out one or more of the following steps:
(i) removal of any carboxyl blocking group;
(ii) converting the product to a pharmaceutically acceptable salt or ester thereof.

2. A process as claimed in claim 1 wherein $R^3$ is phenyl, optionally substituted with $C_{1-6}$ alkyl, $C_{1-6}$ alkoxy, halogen, or nitro.

3. A process as claimed in claim 1 or claim 2 wherein $R^3$ is phenyl or *p*-methylphenyl.

4. A process as claimed in any one of claims 1 to 3 wherein R is 3-thienyl.

5. A process as claimed in any one of claims 1 to 4 for the preparation of 6,$\beta$-(2-carboxy-2-thien-3'-ylacetamido)-6,$\alpha$-methoxypencillanate.

6. A process as claimed in any one of claims 1 to 5 wherein the source of mercuric ions is mercuric acetate.

**Revendications**

1. Procédé pour la preparation d'un dérivé de péname de formule (I):

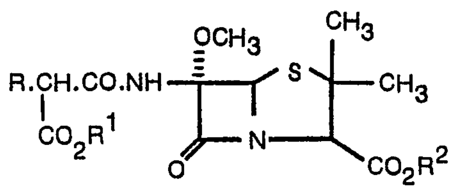

(I)

dans laquelle R est un groupe phényle, p-hydroxyphényle, p-alcoyl($C_{1-6}$)carbonyloxyphényle ou 2- ou 3-thiényle, $R^1$ est de l'hydrogène ou un ion de formation d'un sel pharmaceutiquement acceptable ou un radical de formation d'un ester et $R^2$ représente de l'hydrogène ou un ion de formation d'un sel pharmaceutiquement acceptable ou un radical de formation d'un ester hydrolysable in vivo, caractérisé en ce qu'on fait réagir un composé de formule (II):

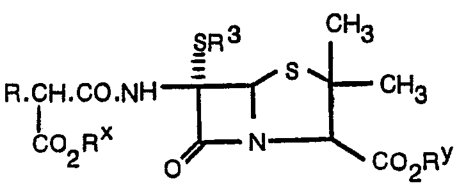

(II)

dans laquelle R est tel que défini dans le cas de la formule (I) ci-dessus, $R^x$ représente un radical de formation d'un ester, $R^y$ représente de l'hydrogène, un radical de formation d'un sel ou un groupe de blocage du carboxyle, et $R^3$ représente un groupe aryle; avec du méthanol en présence de: (a) des ions argent, thallium ou mercurique; ou (b) du chlore ou une base; puis si nécessaire on effectue un ou plusieurs des stades opératoires ci-après:
(i) élimination de tout groupe de blocage du carboxyle;
(ii) conversion du produit en un sel pharmaceutiquement acceptable ou en un ester de celui-ci.

2. Procédé suivant la revendication 1, caractérisé en ce que $R^3$ est un groupe phényle facultativement substitué par un groupe alcoyle en $C_{1-6}$, alcoxy en $C_{1-6}$, halogène ou nitro.

3. Procédé suivant la revendication 1 ou 2, caractérisé en ce que $R^3$ est un groupe phényle ou p-méthylphényle.

4. Procédé suivant l'une quelconque des revendications, 1 à 3, caractérisé en ce que R est un groupe 3-thiényle.

5. Procédé suivant l'une quelconque des revendications 1 à 4, pour la préparation de 6,$\beta$-(2-carboxy-2-thién-3'-ylacétamido)-6,$\alpha$-méthoxypénicillanate.

6. Procédé suivant l'une quelconque des revendications 1 à 5, caractérisé en ce que la source d'ions mercuriques est de l'acétate mercurique.

**Patentansprüche**

1. Ein Verfahren zur Herstellung eines Penamderivates der Formel (I):

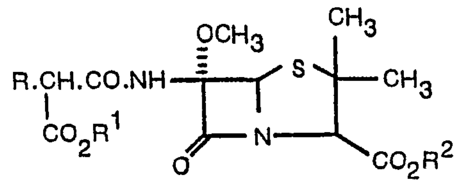

(I)

in welcher R Phenyl, p-Hydroxyphenyl, p-$(C_{1-5})$-Alkylcarbonyloxyphenyl oder 2- oder 3-Thienyl ist, $R^1$ Wasserstoff ist oder ein pharmakologisch annehmbares salzbildendes Ion oder eine esterbildender Rest ist, und $R^2$ Wasserstoff oder ein pharmakologisch annehmbares salzbildendes Ion oder ein in vivo hydrolysierbarer esterbildender Rest ist, *gekennzeichnet durch* das Umsetzen einer Verbindung der Formel(II):

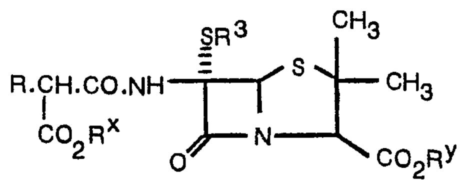

(II)

in welcher R wie in bezug auf die vorstehende Formel (I) definiert ist, $R^x$ einen esterbildenden Rest bedeutet, $R^y$ Wasserstoff, einen salzbildenden Rest oder eine carboxylblockierende Gruppe bedeutet und $R^3$ eine Arylgruppe bedeutet, mit Methanol in Gegenwart von entweder (a) Silber-, Thallium- oder Quecksilber(II)ionen oder (b) Chlor und einer Base und anschließend, falls notwendig, Durchführung von einem oder mehreren der folgenden Verfahrensschritte:
(i) Entfernen von irgendwelchen vorhandenen carboxylblockierenden Gruppen;
(ii) Umwandeln des Produktes in ein pharmakologisch annehmbares Salz oder einen pharmakologisch annehmbaren Ester derselben.

2. Ein Verfahren wie in Anspruch 1 beansprucht, in welchem $R^3$ Phenyl, welches gegebenenfalls mit $C_{1-6}$-Alkyl, $C_{1-6}$-Alkoxy, Halogen oder Nitrogruppe substituiert ist, bedeutet.

3. Ein Verfahren wie in Anspruch 1 oder Anspruch 2 beansprucht, in welchem $R^3$ Phenyl oder p-Methylphenyl ist.

4. Ein Verfahren wie in irgendeinem der Ansprüche 1 bis 3 beansprucht, in welchem R 3-Thienyl ist.

5. Ein Verfahren wie inirgendeinem der Ansprüche 1 bis beansprucht zur Herstellung der Verbindung 6,β-(2-Carboxy-2-thien-3'-ylacetamido)-6,α-methoxy nicillanat.

6. Ein Verfahren wie in irgendeinem der Ansprüche 1 bis 5 beansprucht, in welchem die Quelle für Quecksilber(II)ionen Mercuriacetat ist.